# EUROPÄISCHE PATENTANMELDUNG

(11) **EP 2 163 235 A1**
(43) Veröffentlichungstag der Anmeldung: **17.03.2010**
(21) Anmeldenummer: 08164324.9
(22) Anmeldetag: 15.09.2008
(51) Int. Cl.: A61K 6/083

(54) **Dentalmaterialien mit hohem Biegemodul**

(71) Anmelder: Ivoclar Vivadent AG, 9494 Schaan (LI)
(72) Erfinder: Zappini, Gianluca, 38069, Torbole sui Garda (IT)
(74) Vertreter: Fitzner, Uwe

(57) **Zusammenfassung**

Die vorliegende Erfindung betrifft niedrigviskose Komposite mit einem Biegemodul von mindestens 18 GPa und einer Biegefestigkeit von mindestens 90 MPa, enthaltend
a) Aluminiumoxid mit einer mittleren Teilchengröße von 1 bis 50 µm in Kombination mit mindestens einem weiteren Füllstoff mit einer mittleren Teilchengröße von 0,5 bis 5 µm und
b) eine Monomerenmischung

sowie ein Verfahren zur Herstellung von Zahnrestaurationen und die Verwendung der Komposite.

## Beschreibung

Alle in der vorliegenden Anmeldung zitierten Dokumente sind durch Verweis vollumfänglich in die vorliegende Offenbarung einbezogen (= incorporated by reference in their entirety).

Die vorliegende Erfindung betrifft polymerisierbare Dentalmaterialien mit hohem Biegemodul.

Insbesondere betrifft die vorliegende Erfindung niedrigviskose Komposite mit einem Biegemodul von mindestens 18 GPa und einer Biegefestigkeit von mindestens 90 MPa.

Diese Komposite enthalten einen ersten Füllstoff mit einer mittleren Teilchengröße von 1 bis 50 µm in Kombination mit mindestens einem weiteren Füllstoff mit einer mittleren Teilchengröße von 0,5 bis 5 µm und einer Monomerenmischung. Als besonders bevorzugter erster Füllstoff hat sich Aluminiumoxid herausgestellt.

### Stand der Technik:

Polymerisierbare organische Kunststoffe zur Verwendung als Dentalmaterial sind seit den 30er-Jahren bekannt und insbesondere in der US 3,066,112 wurde die Verwendung eines der bekanntesten Dentalmonomere (Bis-GMA) beschrieben. Aufgrund der mechanischen Eigenschaften der erhaltenen Polymere sind diese allein nicht anwendbar. Eine Verbesserung der Eigenschaften kann durch Füllstoffe verbessert werden. Ein Überblick über die Entwicklung solcher Komposite wird u.a. gegeben in K.-J. M. Söderholm, "Posterior Composite Resin Dental Restorative Materials", S. 139-159, 3M Company, 1985; G. Ott, "Composit-Füllungsmaterialien", Ivoclar Vivadent Report Nr. 5, 1990, U. Salz, "Der gefüllte Zahn - ein komplexes Verbundsystem", Ivoclar Vivadent Report Nr. 7, 1992 und K. Vogel, Füllstofftechnologie", Ivoclar Vivadent Report Nr. 18, S. 18-28, 2007.

Abhängig von der Verwendung der (aus den Hauptbestandteilen Monomer, Füllstoff und Initiator für die Polymerisation bestehenden) Kompositmaterialien sind die einzelnen Komponenten auszuwählen und zu kombinieren. In den letzten Jahren stand bei der Entwicklung der Komposite neben den Verarbeitungseigenschaften (Handling), dem Polymerisationsschrumpf und den mechanischen Eigenschaften vor allen die Ästhetik im Vordergrund. Hinsichtlich ihrer Viskosität kann man Komposite grob in 2 Gruppen unterteilen: Einerseits in stopfbare (hochviskose) Komposite und anderseits fließfähige (niedrigviskose) Komposite (Flowables). Durch den in den Flowables höheren Monomeranteil sind die erreichbaren mechanischen Eigenschaften, insbesondere der Biegemodul, eher als gering einzuschätzen, die Viskosität ist jedoch deutlich niedriger als bei den hochgefüllten Komposits.

Es hat sich jedoch gezeigt, dass es wirtschaftlicher sein kann, auch Komposite für ein eher begrenztes Anwendungsgebiet herzustellen, als es für die sog. Universalkomposits zutrifft.

Bei der Reparatur von devitalen Zähnen werden häufig Stifte in den Wurzeln befestigt, und auf diesen dann die Zahnkrone aufgebaut. Um diese Stifte einsetzen zu können, sind die Wurzelkanäle entsprechend zu präparieren, also aufzubohren und zu glätten. Durch die Krümmung der Wurzelkanäle geht beim Aufbohren natürlich viel gesundes Zahnmaterial verloren, oder man belässt die Krümmung der Wurzel und setzt dünnere oder flexiblere Stifte ein. Insbesondere bei dünnen Stiften kommt es nun darauf an, dass zur Befestigung und zum Aufbau ein Komposit zur Verfügung gestellt werden kann, das die erforderlichen hohen mechanischen Festigkeiten aufweist und sich in den dünnen Wurzelkanälen gut applizieren lässt.

Aus dem Stand der Technik, z.B. JP 1026506, JP 61241303, JP 63303906, JP 58069805, US 6,709,271 B2, US 6,837,712 B2, US 6,696,507 B2, US 6,620,861 B1, US 6,395,803 B1, US 5,865,623, US 5,847,025, US 5,710,194, US 5,192,815, US 4,978,640, sind zwar eine Reihe von Dokumenten bekannt, die die Verwendung von Aluminiumoxid als Füllstoff in Dentalkompositen bzw. Komposite mit guten Eigenschaften beschreiben, jedoch wurde im Stand der Technik nichts offenbart, was die Verwendung der Aluminiumoxidpartikel in einer besonderen Korngröße bei gleichzeitiger Erhöhung bzw. Hochhaltung der mechanischen Festigkeitswerte und vergleichsweise geringer Viskosität der Komposite nahe legt.

Wurzelkanalstifte werden in endodontisch behandelten nicht-vitalen Zähnen verwendet, die eine extensive koronale Schädigung aufweisen. Diese Art von Therapie nennt man postendodontische Behandlung.

In früherer Zeit ging man davon aus, dass bei der endodontischen Behandlung die Wurzelkanalstifte die Wurzelkanalstruktur, die vorher behandelt wurde, verstärken. Heutzutage dienen die Wurzelkanalstifte dazu, eine feste und verlässliche Verankerung für den Stumpfaufbau zur Verfügung zu stellen. Ein Wurzelkanalstift ist bei einer schweren Zerstörung der koronalen Zahnstruktur nötig.

Bis vor kurzem wurden Wurzelkanalstifte noch aus rostfreiem Stahl oder Titan hergestellt. Angesichts dessen, dass mehr und mehr Komposite und keramische Materialien für den Stumpfaufbau verwendet werden, gewinnen ästhetische Überlegungen immer mehr Einfluss auf die Wahl des Stiftmaterials. Dunkle Metallstifte scheinen durch die Komposite und transluzenten keramischen Materialien hindurch, wodurch das natürliche Erscheinungsbild der Restaurationen beeinträchtigt wird. Dies hat zu der Einführung von Stiften geführt, die aus keramischen oder faserverstärkten Kompositen bestehen. Insbesondere faserverstärkte Komposite finden mehr und mehr Akzeptanz.

Üblicherweise besteht die postendodontische Vorgehensweise (direkte Restauration) aus den folgenden Schritten:
- Präparation der koronalen Reststruktur und Entfernung der Wurzelkanalfüllung (Guttapercha) mit Gates-, Peeso- oder Largo-Reibahlen
- Präparation und Aufweitung des Wurzelkanals bis zu der benötigten Tiefe mit Hilfe des korrespondierenden Bohrers
- Testweises Einpassen des endodontischen Stifts, und, falls nötig, Kürzen des Stifts
- Aufbringen von Befestigungskomposit auf den Stift und Einsatz in den Wurzelkanal und Aushärten des Komposites (Lichthärtung oder Selbsthärtung)
- Aufbau des Stumpfs mit einem Stumpfaufbaukomposit

Ein wesentlicher Nachteil dieses Verfahrens besteht in der Aufweitung des Wurzelkanals, denn es besteht die Gefahr der Wurzelperforation und der Schwächung der Wurzelstruktur. Ein Verfahren zur postendodontischen Restauration, welches diesen Schritt nicht mehr enthält, würde die genannten Risikopotenziale eliminieren, es wäre ferner für den Arzt und den Patienten deutlich angenehmer. Idealerweise würde ein solches neues Verfahren auch die Behandlungszeit verkürzen.

Das Problem dabei ist jedoch, dass ohne Aufweitung des Wurzelkanals vorgefertigte, endodontische Stifte nicht passen würden, sofern sie nicht einen deutlich geringeren Durchmesser aufweisen.

Ein geringerer Durchmesser bedeutet allerdings eine geringere Steifigkeit der Stifte und damit der gesamten Rekonstruktion. Eine geringere Steifigkeit wäre der Stabilität der Krone abträglich, so dass die Risiken von Ablösung oder Rissbildung entstehen können.

Theoretisch könnte dieser Mangel an Steifigkeit aufgrund der kleineren Dimensionen kompensiert werden, indem man den Biegemodul des Materials erhöht.
Beispielsweise würde ein Stift mit einem Durchmesser von 0,6 mm in fast jeden Wurzelkanal passen, wohingegen übliche Stifte einen Durchmesser zwischen 1,0 und 2,0 mm aufweisen. Die Verringerung des Durchmessers von 1,0 - 2,0 mm hinunter zu 0,6 mm würde eine 2- bis 3-fache Erhöhung seines Biegemoduls erfordern, um die gleiche Steifigkeit der gesamten Rekonstruktion (Stift plus Stumpfaufbau) zu erreichen. Gebräuchliche faserverstärkte Kompositstifte basieren auf Glas- oder Quarzfasern und weisen einen Biegemodul von ungefähr 40 GPa auf. Dies bedeutet, dass das Material, welches für einen entsprechend dünneren Stift eingesetzt wird, einen Biegemodul von mehr als 80 - 120 GPa aufweisen muss. Gemäß dem aktuellen Stand der Technik auf dem Gebiet der Fasertechnologie, werden solche Eigenschaften jedoch nur erreicht von Kohlenstofffasern, kristallinen Polymerfasern und Keramikfasern (SiC, Al₂O₃). Aufgrund ihrer Färbung (schwarz oder gelb) und ihrer hohen Kosten sind diese Werkstoffe jedoch für diese Anwendung nicht attraktiv. Eine weitere Möglichkeit wäre die Verwendung von Metallstiften - diese sind jedoch unästhetisch und können Allergien auslösen.

Andererseits wird die Steifigkeit der Rekonstruktion aus Stift und Stumpfaufbau ebenfalls durch das Stumpfaufbaumaterial bestimmt. Theoretisch kann die verminderte Steifigkeit des Stifts dadurch kompensiert werden, dass der Biegemodul des Befestigungs- und Stumpfaufbaumaterials auf 20 GPa oder mehr erhöht wird.

Normalerweise weisen Aufbaukomposite einen Biegemodul von 6 bis 15 GPa auf, die Befestigungsmaterialien hingegen nur im Bereich von 3 bis 8 GPa. Es existieren zwar schon einige Dentalkomposite mit Biegemodul-Werten von 18 GPa oder mehr, diese weisen jedoch eine zu hohe Konsistenz auf und können nicht als Befestigungsmaterial innerhalb des Wurzelkanals verwendet werden.

Zusammenfassend sind aus dem Stand der Technik Dentalkomposite bekannt, deren Biegemodul bis 20 GPa beträgt. Es sind andererseits auch Komposite mit niedrigen Viskositäten (sogenannte Flowables) bekannt. Dabei ist es auch bekannt, daß die Komposite mit einem hohen Biegemodul immer eine hohe Viskosität (Eindringtiefe kleiner 1 mm) aufweisen. Dem gegenüber haben Flowables eine niedrige Viskosität (Eindringtiefe 5 bis 20 mm), jedoch ist hier der Biegemodul nur im Bereich von kleiner 10 GPa.

Niedrigviskose Komposite mit gleichzeitig hohem Biegemodul sind nicht aus dem Stand der Technik bekannt.

Es wäre also wünschenswert, ein Dentalkomposit zur Verfügung zu haben, das ein Biegemodul von mehr als 20 GPa und gleichzeitig eine Konsistenz aufweist, die seine Verwendung als endodontisches Befestigungsmaterial erlaubt, also eine Eindringtiefe von mehr als 5 mm aufweist, welches auch gleichzeitig als Stumpfaufbaukomposit eingesetzt werden kann.

### Aufgabe:

Die Aufgabe der vorliegenden Erfindung besteht demgemäß darin, Dentalkomposite bereitzustellen, welche die o.g. Nachteile nicht aufweisen. Insbesondere besteht die Aufgabe der vorliegenden Erfindung darin, Dentalkomposite bereitzustellen, die einen hohen Biegemodul aufweisen und gleichzeitig gute Fließeigenschaften haben.
Diese Dentalkomposite sollen sowohl als Stumpfaufbaukomposite als auch als Befestigungsmaterialien verwendbar sein.

### Lösung:

Diese Aufgabe wird gelöst durch niedrigviskose Komposite, die einen Biegemodul von mindestens 18 GPa und eine Biegefestigkeit von mindestens 90 MPa aufweisen, enthaltend einen ersten Füllstoff mit einer mittleren Teilchengröße von 1 bis 50 µm in Kombination mit mindestens einem weiteren Füllstoff mit einer mittleren Teilchengröße von 0,5 bis 5 µm und einer Monomerenmischung.

### Begriffsdefinitionen:

Die Bestimmung der Eindringtiefe erfolgt im Rahmen der vorliegenden Erfindung in Anlehnung an die ASTM D 1321 ("Standard Test Method for Needle Penetration of Petroleum Waxes"), wobei ein Penetrometer PNR 10, ein Flachkopfzylinder von 4 mm Durchmesser mit einem Gewicht von 17,6 g verwendet wird. Nach einer Messzeit von einer Sekunde wird die Penetration (Eindringtiefe) gemessen.
Im Rahmen dieser Erfindung werden unter hochviskosen Kompositen solche mit einer Eindringtiefe kleiner 1 mm, unter mittelviskosen Kompositen solche mit einer Eindringtiefe von 1 bis 5 mm und unter niedrigviskosen Kompositen solche mit einer Eindringtiefe größer 5 mm verstanden.

Die Eigenschaften "niedrigviskos" und "gute Fließeigenschaften" werden in der Erfindung synonym verwendet, die Einteilung erfolgt dabei nach obiger Einteilung der Komposite hinsichtlich der Eindringtiefe, bestimmt mit einem Penetrometer.

Unter den Begriffen Biegemodul bzw. Biegefestigkeit werden die nach DIN EN ISO 178 ("Kunststoffe - Bestimmung der Biegeeigenschaften") definierten Materialeigenschaften verstanden; die Messung erfolgt hierbei nach der Dentalnorm ISO 4049 ("Zahnheilkunde - Füllungs-, restaurative und Befestigungskunststoffe"). In dieser beschriebenen Messung werden beide Materialeigenschaften gleichzeitig gemessen und auch angegeben.

Die Bestimmung der Teilchengröße kann im Rahmen der vorliegenden Erfindung nach verschiedenen Verfahren erfolgen, diese sind u.a. in Ullmanns Encyklopädie der technischen Chemie, Band 2 (1988), Unit Operations, ausführlich beschrieben, z.B. Windsichten, Klassieren, Flotation, elektrostatische oder auch magnetische Trennungsverfahren. Im für die vorliegende Erfindung relevanten Größenbereich sind die Ergebnisse der einzelnen Methoden vergleichbar, so dass die verschiedenen Bestimmungsmethoden im Rahmen der vorliegenden Erfindung als äquivalent anzusehen sind.

Im Rahmen der vorliegenden Erfindung sind alle Mengenangaben, sofern nicht anders angegeben, als Gewichtsangaben zu verstehen.

Im Rahmen der vorliegenden Erfindung bedeutet der Begriff "Raumtemperatur" eine Temperatur von 23°C. Temperaturangaben sind, soweit nicht anders angegeben, immer in Grad Celsius (°C).

Sofern nichts anderes angegeben wird, werden die angeführten Reaktionen bzw. Verfahrensschritte bei Normaldruck (Atmosphärendruck) durchgeführt.

Im Rahmen der vorliegenden Erfindung werden unter dem Begriff "Photoinitiator", soweit nicht anders angegeben, sowohl Photosensibilisatoren als auch Photoinitiatoren im engeren Sinne verstanden.

Im Rahmen der vorliegenden Erfindung umfasst der Begriff "Komposite" physikalische und/oder chemische Mischungen bzw. Verbindungen von Monomeren, Polymeren, Copolymeren oder Gemischen von Monomeren, Polymeren bzw. Copolymeren mit mindestens 10 Gew.-% an einem oder mehreren anorganischen Stoffen. Anorganische Stoffe, die enthalten sein können, sind bevorzugt Pigmente und Füllstoffe.

Der Ausdruck (Meth)acryl- soll im Rahmen der vorliegenden Erfindung sowohl Methacryl- als auch Acryl- bzw. Mischungen beider umfassen.

### Detaillierte Beschreibung:

Im Rahmen der vorliegenden Erfindung wurde überraschenderweise gefunden, dass die Kombination von ausgewählten Füllstoffen und speziellen Monomeren die Herstellung von Kompositen erlaubt, welche einen hohen Biegemodul und gleichzeitig eine geringere Viskosität aufweisen, so dass sie als Stumpfaufbaumaterial und als Befestigungskomposit eingesetzt werden können.

Demgemäß sind ein Gegenstand der vorliegenden Erfindung niedrigviskose Komposite, die ein Biegemodul von mindestens 18 GPa und eine Biegefestigkeit von mindestens 90 MPa aufweisen, enthaltend einen ersten Füllstoff mit einer mittleren Teilchengröße von 1 bis 50 µm in Kombination mit mindestens einem weiteren Füllstoff mit einer mittleren Teilchengröße von 0,5 bis 5 µm und einer Monomerenmischung. Im Rahmen der vorliegenden Erfindung hat sich Aluminiumoxid besonders bevorzugt als erster Füllstoff herausgestellt.

Das erste wesentliche Element der erfindungsgemäßen Komposite der erste Füllstoff, insbesondere Aluminiumoxid, mit einer mittleren Teilchengröße von 1 bis 50 µm, bevorzugt 5 bis 40 µm, insbesondere bevorzugt 10 bis 25 µm.
Dabei können beispielsweise handelsübliche Aluminiumoxide eingesetzt werden, wie sie z.B. unter der Bezeichnung MKE Edelkorund weiss (Eisenwerk Würth GmbH, D-Bad Friedrichshall), Martoxid (Martinswerk GmbH, D-Bergheim) oder Almatis Alumina (Almatis GmbH, D-67065 Ludwigshafen) erhältlich sind. Die Aluminiumoxide können sphärisch oder auch anders geformt sein. Bevorzugt sind sie durch Mahlen erzeugt, also nicht sphärisch.
Die Aluminiumoxide können in einer bevorzugten Ausgestaltung der vorliegenden oberflächenmodifiziert sein. Dadurch wird einerseits eine gute Einbindung in die Polymerbasis gewährleistet und andererseits eine niedrige Viskosität und eine hohe Biegefestigkeit erreicht.
Die Oberflächenmodifizierung kann nach dem Stand der Technik mit Silanen, mit Anhydriden, mit (Meth)acrylatgruppen oder bevorzugt mit radikalisch polymerisierbaren Monomeren erfolgen, die über saure Gruppen verfügen. Als saure Gruppen der Monomere sind Phosphat-, Phosphonsäure-, Sulfonsäure und/oder Carbonsäuregruppen bevorzugt. Besonders bevorzugt ist die Oberflächenmodifizierung mit phosphatgruppenhaltigen Methacrylaten und ganz besonders bevorzugt mit Methacryloyloxydecyl-dihydrogenphosphat

Das oben beschriebene Aluminiumoxid wird erfindungsgemäß mit mindestens einem weiteren Füllstoff mit einer mittleren Partikelgröße von 0,5 bis 10 µm, insbesondere 0,5 bis 2 µm kombiniert.
Als weitere Füllstoffe sind die in der Dentaltechnik gebräuchlichen Füllstoffe wie Glas- bzw. Glaskeramikfüllstoffe, Quarz, Kompositfüllstoffe, pyrogene Kieselsäuren geeignet. Dabei kann auch der weitere Füllstoff ein Aluminiumoxid oder ein weiteres Metalloxid (wie z.B. Titanoxid, Zirkonoxid) sein - allerdings mit entsprechend geringeren mittleren Partikelgrößen.

Bevorzugt ist der Einsatz eines oberflächenmodifizierten Glasfüllstoffs als weiterem Füllstoff, wobei die Oberflächenmodifizierung bevorzugt über Silane erfolgt. Hierzu können übliche, käuflich erhältliche Silanisierungsmittel wie Vinyltriethoxysilan, Vinyltrimethoxysilan, Vinyl-tris(2-methoxyethoxy)silan, gamma-Methacryloxypropyl-tri-methoxy-silan (Silan A-174), gamma-Methacryloxypropyl-tris(2-methoxyethoxy)silan, gamma-Glycidoxypropyltrimethoxysilan, Vinyltriacetoxysilan, gamma-Mercaptopropyltrimethoxysilan, gamma-Aminoprpyltriethoxysilan, N-beta(aminoethyl)-gamma-aminopropyltrimethoxysilan (Fa. Union Carbide) eingesetzt werden. Das A-174 ist dabei besonders bevorzugt.
Als Alternative zum Aluminiumoxid sind weitere erste Füllstoffe verwendbar, das Kriterium hierfür ist, dass diese einen E-Modul von mehr als 200 GPa aufweisen. Beispiel hierfür sind z.B. ZrO₂ und TiO₂ und übliche Carbide, Diboride oder Nitride wie TaC, TiC, WC, ZrC, VC, NbC, SiC, B₄C₃, TiB₂, ZrB₂, MgB₂, Si₃N₄, TiN, AIN.

In einer bevorzugten Variante werden zusätzlich röntgenopake Füllstoffe zugegeben. Als röntgenopake Füllstoffe werden bevorzugt Oxide und Salze von Schwermetallen eingesetzt, besonders bevorzugt werden die röntgenopake Füllstoffe ausgewählt aus der Gruppe bestehend aus Ytterbiumtrifluorid, nanopartikulärem Tantal(V)-oxid , Bariumsulfat, Wismutoxid und Mischungen davon. Besonders bevorzugt ist YbF₃.

Als Monomere für den Einsatz in der Monomerenmischung der vorliegenden Erfindung kommen alle in der Dentaltechnik gebräuchlichen Monomere, insbesondere die radikalisch polymerisierbaren mono- und multifunktionellen (Meth)acrylate, in Betracht.
Als radikalisch polymerisierbare Monomere können mono- oder mehrfach funktionelle (Meth)acrylate bzw. (Meth)acrylamide ((Meth)acrylverbindungen) eingesetzt werden. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden. Mehrfach funktionelle Monomere haben vernetzende Eigenschaften.
Bevorzugte monofunktionelle (Meth)acrylverbindungen sind kommerziell verfügbare monofunktionelle Monomere, wie Methyl-, Ethyl-, Butyl-, Benzyl-, Furfuryl- oder Phenyl(meth)acrylat sowie 2-Hydroxyethyl- oder -propyl(meth)acrylat.
Bevorzugte mehrfach funktionelle (Meth)acrylverbindungen sind Bisphenol-A-di(meth)acrylat, Bis-GMA (ein Additionsprodukt aus Methacrylsäure und Bisphenol-A-diglycidylether), ethoxyliertes Bisphenol-A-di(meth)acrylat, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Butandioldi(meth)acrylat, 1,10-Decandioldi(meth)acrylat oder 1,12-Dodecandioldi(meth)acrylat., vernetzende Pyrrolidone, wie z.B. 1,6-Bis-(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N'-Diethyl-1,3-bis-(acrylamido)-propan, 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechlorid synthetisiert werden können.

Weiterhin einsetzbar sind hydrolysestabile Monomere wie hydrolysestabile Mono(meth)acrylate, z.B. Mesitylmethacrylat oder 2-(Alkoxymethyl)acrylsäuren, z.B. 2-(Ethoxymethyl)acrylsäure, 2-(Hydroxymethyl)acrylsäure, N-mono- oder disubstituierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, und N-monosubstituierte Methacrylamide, wie z.B. N-Ethylmethacrylamid oder N-(2-Hydroxyethyl)methacrylamid sowie außerdem N-Vinylpyrrolidon und Allylether. Diese Monomere sind bei Raumtemperatur flüssig und eignen sich daher auch als (Reaktiv)Verdünner.

Ebenfalls einsetzbar sind radikalisch polymerisierbare, säuregruppenhaltige Monomere. Bevorzugte Säuregruppen sind Carbonsäuregruppen, Phosphonsäuregruppen, Phosphatgruppen und/oder Sulfonsäuregruppen, wobei diese Gruppen in der Säureform, als Anhydrid oder in Form eines Esters vorliegen können. Bevorzugt sind dabei Monomere mit Phosphonsäuregruppen oder Phosphatgruppen. Die Monomere können eine oder mehrere acide Gruppen aufweisen, bevorzugt sind Verbindungen mit 1 bis 2 aciden Gruppen.
Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäure bzw. das entsprechende Anhydrid, 10-Methacryloyloxydecylmalonsäure, N-(2-Hydroxy-3-methacryloyloxypropyl)-N-phenylglycin, N-Acryloyl-alpha-asparaginsäure (AAA) und 4-Vinylbenzoesäure.
Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure 2-[4-(Dihydroxyphosphoryl)-2-oxa-butyl]-acrylsäure, 2-[2-Dihydroxyphosphoryl)-ethoxy-methyl]-acrylsäure-2,4,6-trimethyl-phenylester und 2-[2-Dihydroxyphosphoryl)-ethoxy-methyl]-acrylsäure-ethylester (DPEAE).
Bevorzugte acide polymerisationsfähige Phosphorsäureester sind 2-Methacryloyloxypropylmono- und -dihydrogenphosphat, 2-Methacryloyloxyethylmono- und -dihydrogenphosphat, 2-Methacryloyloxyethyl-phenylhydrogenphosphat, Dipentaerythritol-pentamethacryloyloxyphosphat, 10-Methacryloyloxydecyl-dihydrogenphosphat (MDP), Dipentaerythritolpentamethacryloyloxyphosphat, Phosphorsäuremono-(1-acryloylpiperidin-4-yl)-ester, 6-(Methacrylamido)hexyldihydrogenphosphat, 1,3-Bis-(N-acryloyl-N-propyl-amino)-propan-2-yl-dihydrogenphosphat und 1,3-Bis-(methacrylamido)-propan-2-yl-dihydrogenphosphat (BMPP).
Bevorzugte polymerisationsfähige Sulfonsäuren sind Vinylsulfonsäure, 4-Vinylphenylsulfonsäure oder 3-(Methacrylamido)propylsulfonsäure.
Als mono- oder multifunktionelles Monomer können auch polymerisierbare Verbindungen zum Einsatz kommen, die eine antimikrobielle Wirkung aufweisen, wie z.B. 12-Methacryloyloxydodecylpyridiniumbromid, besonders bevorzugt sind so genannte Makromere, die einen polymeren Spacer zwischen der polymerisierbaren und der antimikrobiell wirksamen Gruppe enthalten.
Ebenfalls einsetzbar sind spezielle Monomere, wie z.B. die in der DE 10 2007 035 734 A1 in den Absätzen [0021] und [0022] beschriebenen polymerisierbaren Calix[n]arene.

Besonders gute Ergebnisse werden enthalten, wenn die Monomeren der Monomerenmischung aus zwei oder mehr Monomeren aus der Gruppe bestehend aus Urethandi(meth)acrylat, ethoxyliertem Bisphenol-A-di(meth)acrylat, Di-trimethylolpropan Tetra(meth)acrylat, Decandioldi(meth)acrylat, Tricyclodecandimethanoldi(meth)acrylat, Tetrahydrofurfuryl(meth)acrylat, Hydroxyethyl(meth)acrylat, Dimethyl(meth)acrylamid, Dimethylaminoethyl(meth)acrylat ausgewählt werden, wobei es bevorzugt ist die Monomere aus der Gruppe bestehend aus Urethandimethacrylat, ethoxyliertem Bisphenol-A-dimethacrylat, Ditrimethylolpropantetraacrylat, Decandioldimethacrylat, Tricyclodecandimethanoldimethacrylat, Tetrahydrofurfurylmethacrylat, Hydroxyethylmethacrylat, Dimethylacrylamid, Dimethylaminoethylmethacrylat auszuwählen.

In einer insbesondere bevorzugten Variante der vorliegenden Erfindung sind in der Monomerenmischung, insbesondere wenn diese aus Monomeren der soeben genannten Gruppen besteht, als reaktive Verflüssiger (Reaktivverdünner) zwischen 5 und 30, bevorzugt zwischen 10 und 25 Gew.-% hydrolysebeständige (Meth)acrylamide und/oder Amino(meth)acrylate enthalten.

Im Rahmen der vorliegenden Erfindung werden für die radikalische Polymerisation die üblichen Initiatoren für die Licht- und Selbsthärtung eingesetzt. Als Polymerisationstyp sind hierbei die Selbsthärtung und die Dualhärtung bevorzugt, es ist aber auch eine reine Lichthärtung (Photopolymerisation) möglich.
Bei der Lichthärtung (Photopolymerisation) erfolgt die Initiierung der Aushärtung durch eine Radikalbildung der Photoinitiatoren selbst (Norrish-Typ I) oder mit zusätzlich verwendeten Coinitiatoren (Norrish-Typ II).
Unter Selbsthärtung wird im Rahmen der vorliegenden Erfindung verstanden, dass die Aushärtung spontan, ohne weitere Energiezufuhr (wie Licht oder Wärme) erfolgt. Hier werden immer Kombinationen aus Initiatoren und Aktivatoren oder die bekannten Redoxsyteme verwendet.
Unter Dualhärtung wird im Rahmen der vorliegenden Erfindung eine Kombination von Selbsthärtung und Lichthärtung verstanden.
Diese Zusammenhänge sind dem Fachmann bekannt, dennoch soll der folgende Absatz dem besseren Verständnis dienen.
Bei der Selbsthärtung erfolgt die Einleitung der Polymerisation ohne äußere Energiezufuhr. Sie wird erreicht durch Systeme von Aktivatoren (Reduktionsmittel) und Katalysatoren/Initiatoren (Oxidationsmittel), auch bekannt als Redox-Systeme, die sich getrennt in zwei Komponenten des Füllungsmaterials befinden und beim Anmischen beider die Reaktion in Gang setzen.
Im Rahmen der vorliegenden Erfindung für die Selbsthärtung einsetzbare Redoxsysteme basieren auf einem Oxidationsmittel, insbesondere Peroxid oder Hydroperoxid, und einem Reduktionsmittel. Prinzipiell können im Rahmen der vorliegenden Erfindung alle üblichen Redoxsysteme mit den bekannten Oxidationsmitteln und Reduktionsmitteln eingesetzt werden.

Erfindungsgemäß gut einsetzbare Oxidationsmittel sind Kobalt(III)chlorid, tert.-Butylhydroperoxid, Eisen(III)chlorid, Perborsäure und ihre Salze und Salze mit Permaganat- oder Persulfatanionen und insbesondere Dibenzoylperoxid, Dilaurylperoxid, höchst bevorzugt Dibenzoylperoxid.
Erfindungsgemäß einsetzbare Reduktionsmittel sind Kobalt(II)chlorid, Eisen(II)chlorid, Eisen(II)sulfat, Hydrazin, Oxalsäure, Thioharnstoff und Salze mit Dithionit- oder Sulfatanionen.
Erfindungsgemäß besonders gut einsetzbare Reduktionsmittel sind tertiäre aromatische Amine, wie z.B. N,N-Diethanol-p-toluidin, N,N-Dimethyl-p-toluidin, N,N-Dihydroxyethyl-p-toluidin, N,N-Diethyl-3,5-di-tert.-butylanilin oder N,N-Dimethyl-sym.-xylidin, Ascorbinsäure, p-Toluolsulfinsäure oder Trimethylbarbitursäure, insbesondere N,N-Dimethyl-p-toluidin, N,N-Dimethyl-sym.-xylidin und Diethyl-3,5-di-tert.-Butylanilin.
Insbesondere bevorzugt werden im Rahmen der vorliegenden Erfindung für die Selbsthärtung Benzoylperoxid bzw. Diethyl-3,5-di-tert.-Butylanilin eingesetzt.

Geeignete Photoinitiatoren sind z.B. Dibenzoyldiethylgermanium, Dibenzoyldimethylgermanium, Benzophenon, Benzoin sowie Derivate, insbesondere Benzoinether, Dialkylbenzilketale, Dialkoxyacetophenone, Acyl- der Bisacylphosphinoxide, alpha-Diketone oder deren Derivate wie 9,10-Phenanthrenchinon, Diacetyl, Furil, Anisil, 4,4'-Dichlorbenzil und 4,4'-Dialkoxybenzil, 2,2-Dimethoxy-2-phenyl-acetophenon und Campherchinon.

Als Photoinitiator wird im Rahmen der vorliegenden Erfindung bevorzugt Campherchinon eingesetzt. Als Coinitiator für die Lichthärtung wird bevorzugt 4-Dimethylaminobenzoesäureethyester (EMBO) eingesetzt.
Als Photoinitiatoren im Rahmen der vorliegenden Erfindung sind aber auch alle anderen üblichen Photoinitiatoren verwendbar, gegebenenfalls auch im Kombination miteinander.
Beispiele für besonders geeignete Photoinitiatoren sind neben Campherchinon und EMBO unter anderem die Acylphosphinoxide, insbesondere 2,4,6-Trimethylbenzoyldiphenylphosphinoxid (Lucirin TPO) oder Bis(2,6-dichlorbenzoyl)-4-N-propylphenylphosphinoxid.

Schließlich lassen sich den erfindungsgemäßen Kompositen im Bedarfsfalle weitere Additive zusetzen, wie z.B. Stabilisatoren, UV-Absorber, Aromastoffe, Fluoridionen abgebende Additive, optische Aufheller, Weichmacher, Farbstoffe oder Pigmente.
Ein bevorzugter UV-Absorber dabei ist 2-Hydroxy-4-methoxybenzophenon, bevorzugte Stabilisatoren sind 2,6-Di-tert-butyl-4-cresol und 4-Methoxyphenol.

Sofern die erfindungsgemäßen Komposite durch Selbsthärtung oder Dualhärtung ausgehärtet werden sollen, werden die Bestandteile in eine Basis- und eine Katalysatorpaste, bevorzugt im Volumenverhältnis 1:1 aufgeteilt.
Dazu wird aus der Monomerenmischung und den Füllstoffen eine Ausgangspaste angefertigt, die dann in gleiche Teile aufgeteilt wird. In den ersten Teil, die Katalysatorpaste, wird dann das Oxidationsmittel, gegebenenfalls zusammen mit einem Stabilisator gegeben, in den zweiten Teil, die Basispaste, das Reduktionsmittel und für den Fall der Dualhärtung der Photoinitiator.

In einer bevorzugten Variante der vorliegenden Erfindung bestehen die niedrigviskosen Komposite aus
- 10 bis 40 Gew.-% Monomerenmischung
- 40 bis 70 Gew.-% Aluminiumoxid mit einer mittleren Teilchengröße von 1 bis 50 µm,
- 10 bis 45 Gew.-% mindestens einem weiterem Füllstoff mit einer mittleren Teilchengröße von 0,5 bis 5 µm und
- 0,001 bis 3 Gew.-% Initiatoren und weiteren Additiven,
wobei sich die Prozentangaben auf 100 Gew.-% addieren.

Die erfindungsgemäßen Komposite weisen thixotrope Eigenschaften auf, d.h. bei Einwirkung von Kräften erniedrigen sie ihre Viskosität reversibel; nach Ende der Krafteinwirkung weisen sie wieder die ursprüngliche Viskosität auf.
Sie zeigen eine geringe Viskosität, d.h. eine Eindringtiefe von mehr als 5 mm, gemessen mit der Penetrationsmethode .
Die erfindungsgemäßen Komposite weisen einen Biegemodul von größer als 18 GPa auf. Bevorzugt ist es, wenn sie einen Biegemodul von 18 bis 30 GPa, bevorzugt 20 bis 25 GPa aufweisen.
Dabei weisen sie gleichzeitig eine Biegefestigkeit von 90 bis 300 MPa, bevorzugt 100 bis 180 MPa, auf.

Aufgrund der thixotropen Eigenschaften der erfindungsgemäßen Komposite, d.h. einer Viskositätserniedrigung bei hohen Scherkräften, wie z.B. beim Ausdrücken aus Spritzen, kann sehr leicht ein enger Wurzelkanal gefüllt werden, in den danach ein Stift, z.B. aus Metall, Keramik oder faserverstärktem Komposit, eingesetzt und durch Aushärten des Komposits befestigt wird.
Durch den hohen Biegemodul der erfindungsgemäßen Komposite ist ein stabiler Aufbau, der zur Unterstützung der Krone dient, gewährleistet.

Die Komposite der vorliegenden Erfindung eignen sich insbesondere für die Verwendung
a) als Füllung von Zahnwurzelkanälen zur Befestigung von Stiften,
b) als Aufbaumaterial, z.B. auf Stifte.

Die erfindungsgemäßen Komposite weisen eine Reihe von Vorteilen auf, von denen die wichtigsten die folgenden sind:
- Das Komposit kann sowohl für die Befestigung von Stiften, als auch für die Konstruktion des Aufbaus verwendet werden - ein Produkt, statt wie bisher zwei Produkte.
- Die Stabilität des Aufbaus ist auch bei der Verwendung von sehr dünnen Stiften gewährleistet; es ist nicht notwendig, den Wurzelkanal mittels Bohrer zu erweitern; die Behandlung ist schonender für die Zahnsubstanz, das Risiko von Wurzelperforation aufgrund von Bohrungsfehler ist geringer als bei bisherigen Verfahren.

Gegenstand der vorliegenden Erfindung ist auch ein Verfahren zur Zahnrestauration, dass die folgenden Schritte umfasst:
a) Präparation der koronalen Reststruktur und Entfernung der Wurzelkanalfüllung (Guttapercha) mit Gates-, Peeso- oder Largo-Reibahlen.
b) Präparation des Wurzelkanals bis zu der benötigten Tiefe mit Hilfe des korrespondierenden Bohrers.
c) Testweises Einpassen des endodontischen Stifts,
d) falls nötig, Kürzen des Stifts,
**dadurch gekennzeichnet, dass** ein Komposit gemäß der vorliegenden Erfindung eingesetzt wird.

Aufgrund des hervorragenden Eigenschaftsprofils sind die erfindungsgemäßen Komposite auch dazu geeignet, in anderen Bereichen als der Zahnmedizin eingesetzt zu werden.

In einer Variante der vorliegenden Erfindung sind die Komposite frei von Polypropylengylcol- oder Polytetramethylenglycol-Dimethacrylaten.

Die verschiedenen Ausgestaltungen der vorliegenden Erfindung, z.B. diejenigen der verschiedenen abhängigen Ansprüche, können dabei in beliebiger Art und Weise miteinander kombiniert werden.

Die Erfindung wird nun unter Bezugnahme auf die folgenden, nicht limitierenden Beispiele erläutert.

### Beispiele:

In den Beispielen werden folgende Abkürzungen verwendet:

| Abkürzung | Bedeutung |
|---|---|
| V380 | Urethandimethacrylat |
| SR348C | Ethoxyliertes Bisphenol-A-Dimethacrylat |
| SR355 | Di-Trimethylolpropan Tetraacrylat |
| D3MA | Decandiol Dimethacrylat |
| DCP | Tricyclodecan Dimethanol Dimethacrylat |
| THFMA | Tetrahydrofurfuryl Methacrylat |
| HEMA | Hydroxyethyl Methacrylat |
| N,N-DMAA | Dimethyl Acrylamide |
| DMA-EMA | Dimethylamino Ethylmethacrylat |
| MDP | Methacryloyl Oxydecyl Dihydrogenphosphat |
| A-174 | gamma-Methacryloxypropyl-tri-methoxy-Silan |
| F360 | High-grade Korundpulver (Al₂O₃, 12-40 µm) |
| F1000 | High-grade Korundpulver (Al₂O₃, 1-10 µm) |
| GM27884 | Bariumglas Pulver (mittlere Korngröße 1 µm) |
| YbF3 | Ytterbium Trifluoride powder (mittlere Korngröße 0.1 µm) |
| CC | Campherchinon |
| EPD bzw. EMBO | p-Dimethylaminobenzoesäureethylester |
| DABA | Diethyl-3,5-di-tert.-Butylanilin |
| BPO | Benzoylperoxid |
| BHT | Di-tert.-Butyl-p-Kresol |

### Beispiele 1 bis 6:

Für die Beispiele 1 bis 6 wurden die folgenden Zusammensetzungen verwendet, wobei die Zahlenwerte Gew.-% darstellen. Die Herstellung der Komposite erfolgt durch intensives Mischen, z.B. auf einem Kneter, anschließendes Entlüften und das Abfüllen in geeignete Primärverpackungen (Spritzen oder Cavifills).

| (Gew.-%) | 1 | 2 | 3 | 4 | 5 | 6 |
|---|---|---|---|---|---|---|
| V380 | 5.0 | 5.2 | 5.1 | 5.6 | - | - |
| SR348C | 4.6 | - | 4.5 | 5.0 | 3.9 | 5.0 |
| SR355 | - | 3.0 | - | - | - | - |
| D3MA | - | 2.2 | - | - | - | - |
| DCP | - | - | - | - | 4.9 | 6.2 |
| THFMA | 2.0 | - | 2.0 | 2.3 | - | - |
| HEMA | 0.6 | 1.8 | 0.6 | 0.6 | 1.0 | 1.2 |
| N,N-DMAA | 1.6 | 1.7 | - | 1.7 | - | - |
| DMA-EMA | - | - | 1.6 | - | 2.8 | 1.2 |
| MDP | 1.0 | 0.9 | 1.1 | 0.9 | 0.8 | - |
| A-174 | - | - | - | - | - | 2.1 |
| F360 | 56.6 | 48.1 | 56.5 | 40.3 | 55.6 | 50.1 |
| F1000 | 6.9 | 16.8 | 6.9 | 17.2 | 5.5 | - |
| GM27884 | 21.6 | 20.2 | 21.6 | 26.3 | 25.4 | 17.0 |
| YbF3 | - | - | - | - | - | 17.1- |
| CC | 0.05 | 0.05 | 0.05 | 0.05 | 0.05 | 0.05 |
| EPD | 0.05 | 0.05 | 0.05 | 0.05 | 0.05 | 0.05 |
| Summe | 100 | 100 | 100 | 100 | 100 | 100 |

Physikalische Eigenschaften der Zusammensetzungen gemäß den Beispielen 1 bis 6 im Vergleich zu Zusammensetzungen des Standes der Technik, die keine Aluminiumoxid-Füllstoffe enthalten:

| | Beschreibung | Penetrationstiefe | Biegemodul | Biegefestigkeit |
|---|---|---|---|---|
| | | [mm] ** | [GPa] * | [MPa] * |
| Beispiel 1 | Fließfähig (flowable) | 5.2 | 24 | 148 |
| Beispiel 2 | Fließfähig (flowable) | 7.2 | 24 | 147 |
| Beispiel 3 | Fließfähig (flowable) | 6.3 | 21 | 124 |
| Beispiel 4 | Fließfähig (flowable) | 12.8 | 21 | 124 |
| Beispiel 5 | Fließfähig (flowable) | 8.5 | 22 | 100 |
| Beispiel 6 | Fließfähig (flowable) | 12.0 | 20.1 | 120 |
| Tetric Ceram | Hochviskose Paste, | < 0.1 | 10.0 | 120 |
| | Füllungskomposit | | | |
| In Ten-S | Hochviskose Paste, | < 0.1 | 10.5 | 130 |
| | Füllungskomposit | | | |
| Tetric EvoFlow | Fließfähig, Füllungskomposit | 6.7 | 5.1 | 114 |
| Variolink II | Fließfähig, | 6.2 | 8.3 | 115 |
| | Befestigungskomposit | | | |
| Multicore HB | Hochviskose Paste, | < 0.1 | 14 - 18 | 125 - 140 |
| | Stumpfaufbaumaterial | | | |
| Multicore Flow | Fließfähig, | 4.7 | 7.5 - 9.0 | 120 - 135 |
| | Stumpfaufbaumaterial | | | |
| LuxaCore | Fließfähig, | 5.6 | 8.8 | 100 |
| | Stumpfaufbaumaterial | | | |
| Rebilda DC | Fließfähig, | 9.6 | 8.0 | 125 |
| | Stumpfaufbaumaterial | | | |

| | | | | |
|---|---|---|---|---|
| *: gemessen mit dem 3-Punkt Biegetest nach ISO 4049. Ein Durchschnittswert von mindestens sechs Messungen ist angegeben. **: gemessen mit dem Penetrometer PNR 10 mit 4 mm Flachkopfzylinder, 17,6 g. Das Komposit wurde in eine Mulde mit einem Durchmesser von 10 mm und einer Tiefe von 20 mm gegeben. Die Penetration wurde für eine Sekunde vorgenommen. Ein Durchschnittswert von drei Messungen ist angegeben. Die Messung der Eindringtiefe erfolgt dabei in Anlehnung an die ASTM D 1321, wie vorstehend beschrieben. | | | | |

Wie aus den Beispielen 1 bis 6 ersichtlich ist, weisen die erfindungsgemäßen Komposite eine niedrige Viskosität bei gleichzeitig hohem Biegemodul auf. Im Gegensatz dazu weisen die Zusammensetzungen des Standes der Technik niemals beides zusammen auf.

### Beispiel 7:

Für ein selbsthärtendes Komposit wurde die Ausgangspaste aus den folgenden Komponenten hergestellt.

| Komponente: | Gew.-% |
|---|---|
| SR348C | 4.5 |
| DCP | 5.7 |
| HEMA | 1.1 |
| DMA-EMA | 1.4 |
| MDP | 0.4 |
| F360 | 42.7 |
| GM27884 | 14.8 |
| YbF₃ | 29.4 |

Diese Ausgangspaste wurde dann zu gleichen Teilen auf eine Basispaste und eine Katalysatorpaste aufgeteilt und die Initiatoren für die Selbsthärtung zugefügt:

| Basispaste | | | Katalysatorpaste | |
|---|---|---|---|---|
| Komponente | Gew.-% | | Komponente | Gew.-% |
| Ausgangspaste | 98.9 | | Ausgangspaste | 98.9 |
| CC | 0.05 | | BPO | 1.00 |
| EMBO | 0.05 | | BHT | 0.10 |
| DABA | 1.00 | | | |

Beide Pasten hatten eine Penetrationstiefe von 8.8 mm. Nach der Aushärtung wurde ein Biegemodul von 20 GPa und eine Biegefestigkeit von 100 MPa bestimmt.

## Patentansprüche

1. Niedrigviskose Komposite mit einem Biegemodul von mindestens 18 GPa und einer Biegefestigkeit von mindestens 90 MPa, enthaltend
a) einen ersten Füllstoff mit einer mittleren Teilchengröße von 1 bis 50 µm in Kombination mit mindestens einem weiteren Füllstoff mit einer mittleren Teilchengröße von 0,5 bis 5 µm und
b) eine Monomerenmischung.

2. Niedrigviskose Komposite nach Anspruch 1, **dadurch gekennzeichnet, dass** der erste Füllstoff einen E-Modul von mindestens 200 GPa besitzt.

3. Niedrigviskose Komposite nach Anspruch 1, **dadurch gekennzeichnet, dass** der erste Füllstoff Aluminiumoxid ist.

4. Niedrigviskose Komposite nach Anspruch 1, **dadurch gekennzeichnet, dass** der weitere Füllstoff aus der Gruppe bestehend aus Glas, Glaskeramik, Quarz, Komposit, pyrogene Kieselsäuren, Titandioxid, Zirkoniumdioxid und Aluminiumoxid ausgewählt wird.

5. Niedrigviskose Komposite nach Anspruch 1, **dadurch gekennzeichnet, dass** sie aus
10 bis 40 Gew.-% Monomerenmischung
40 bis 70 Gew.-% einem ersten Füllstoff mit einer mittleren Teilchengröße von 1 bis 50 µm,
10 bis 45 Gew.-% mindestens einem weiterem Füllstoff mit einer mittleren Teilchengröße von 0,5 bis 5 µm und
0,001 bis 3 Gew.-% Initiatoren und weiteren Additiven bestehen,
wobei sich die Prozentangaben auf 100 Gew.-% addieren.

6. Niedrigviskose Komposite nach Anspruch 1, **dadurch gekennzeichnet, dass** das Aluminiumoxid und gegebenenfalls auch der weitere Füllstoff oberflächenmodifiziert sind.

7. Niedrigviskose Komposite nach Anspruch 3, **dadurch gekennzeichnet, dass** das Aluminiumoxid eine mittlere Teilchengröße von 5 bis 40 µm, insbesondere bevorzugt 10 bis 25 µm aufweist.

8. Niedrigviskose Komposite nach Anspruch 1, **dadurch gekennzeichnet, dass** sie Photoinitiatoren und/oder Redoxsysteme zur Initiation der Polymerisation enthält.

9. Verfahren zur Zahnrestauration, umfassend die folgenden Schritte:
a) Präparation der koronalen Reststruktur und Entfernung der Wurzelkanalfüllung (Guttapercha) mit Gates-, Peeso- oder Largo-Reibahlen,
b) Präparation des Wurzelkanals bis zu der benötigten Tiefe mit Hilfe der korrespondierenden Reibahle bzw. des korrespondierenden Bohrers,
c) testweises Einpassen des endodontischen Stifts, und,
d) falls nötig, Kürzen des Stifts,
**dadurch gekennzeichnet, dass** ein Komposit gemäß der vorliegenden Erfindung eingesetzt wird.

10. Verwendung der Komposite nach einem der Ansprüche 1 bis 8
a) als Füllung von Zahnwurzelkanälen zur Befestigung von Stiften,
b) als Aufbaumaterial, z.B. auf Stifte.
